# EUROPEAN PATENT SPECIFICATION

(11) **EP 1 520 526 B1**
(45) Date of publication and mention of the grant of the patent: **05.11.2008**
(21) Application number: 04256009.4
(22) Date of filing: 29.09.2004
(51) Int. Cl.: A61B 17/11

(54) **Applier for a surgical device**
Einsetzer für eine chirurgische Vorrichtung
Appareil pour appliquer un dipositif chirurgical

(30) Priority: 30.09.2003 US 507799 P
(43) Date of publication of application: 06.04.2005
(73) Proprietor: ETHICON ENDO-SURGERY, INC., Cincinnati, Ohio 45242 (US)
(72) Inventor: Beaupre, Jean, Cincinnati Ohio 45249 (US)
(74) Representative: Mercer, Christopher Paul

(56) References cited:
- FR-A- 2 245 324
- US-A- 5 478 353
- US-A1- 2003 120 292
- US-A1- 2003 216 749

## Description

### Field of the Invention

The present invention relates, in general, to devices for surgically modifying organs and vessels. More particularly, it relates to anastomosis devices for joining two organs such as, for example, two separate lengths of small bowel to each other, a section of small bowel to the stomach, or the common bile duct to the duodeneum in a procedure called a choledochoduodenostomy.

### Background of the Invention

Creating an anastomosis, or the surgical formation of a passage between two normally distinct vessels, is a critical step of many surgical procedures. This is particularly true of gastric bypass procedures in which two portions of small intestine are joined together and another portion of small intestine is joined to the stomach of the patient. This is also true of surgery to alleviate blockage in the common bile duct by draining bile from the duct to the small intestine during surgery for pancreatic cancer.

For many anastomosis, surgeons use circular staplers, linear staplers, or manual sutures. However, to reduce incision size and to make the surgical process less technically demanding and time consuming, an expandable medical device that deforms to hold tissue portions together when the device is ejected from a constraining enclosure has been described. United States Application 2003/0120292 to Adrian Park et al describes such a device in accordance with the preamble of claim 1.

The expandable medical device disclosed in 2003/0120292 is constrained by a sleeve to an advantageous small-diameter tubular shape. A surgeon applies the expandable medical device by maneuvering the sleeve through the tissue portions requiring anastomosis, moving a nose assembly distally away from the sleeve, and ejecting the device with a ram. Ejecting the device removes the constraint on the device, allowing the device to assume a roughly ring shape. The larger ends of the ring shape hold the two tissue portions together in an effective anastomosis.

The constrained expandable medical device, which may be made of a shape memory material such as nitinol, exerts a force against the inner diameter of the sleeve and tends to warp towards its roughly ring-shaped deployed position. When the device is ejected, the forces generated by the device in transition from a tubular shape to a ring shape urge the expandable medical device distally. This device movement makes surgical control harder to achieve when placing the device through the otomies of two tissue portions requiring anastomosis. Applicants have recognized a need to apply a restraining force to the distal end of the expandable medical device to improve surgical control when applying it. An applier to place the expandable medical device while restraining the tendency of the device to move distally during ejection, and a method of using the applier would be desirable. It would be further advantageous to provide a flexible force element urging the distal nosepiece, or cover, towards the ejecting device as the device is ejected to control distal movement of the device. The present invention provides such an applier.

### Summary of the Invention

In accordance with the present invention there is provided an applier for a surgical device as defined in claim 1 that gives the surgeon improved control when applying the device. The applier includes an enclosure to constrain the device, a cover on the end of the enclosure, a restraining element to prevent proximal movement of the device while the enclosure moves proximally, and a force element connecting the cover to the enclosure. The force element causes the cover and enclosure to retract proximally as one unit, but has enough flexibility to allow the cover to lift off of the enclosure when a second force larger than that generated by the force element is placed on the cover to urge it distally. The force element places a force at a distal end of the surgical device as the applier ejects the surgical device.

### Brief Description of the Drawings

The novel features of the invention are set forth with particularity in the appended claims. The invention itself, however, both as to organization and methods of operation, together with further objects and advantages thereof, may best be understood by reference to the following description, taken in conjunction with the accompanying drawings in which:
Figure 1 is an isometric view of an applier according to an embodiment of the invention containing an expandable medical device.
Figure 2 is an isometric of an applier according to a second embodiment of the invention.
Figure 3 is an isometric view of the applier of Figure 2 with interior portions revealed.
Figure 4 is an isometric view of the distal side of the handle of the applier of Figure 3.
Figure 5 is an isometric view of an applier according to a third embodiment of the invention.
Figure 6 is an isometric view of the interior portions of the embodiment of Figure 5.
Figure 7 is an isometric view of the path retainer on the applier of Figure 5 depicting details of the guide path.
Figure 8 is a cross-sectional view of the embodiment of Figure 1 in the loaded position.
Figure 9 is an isometric view of embodiment of Figure 1 with the device in the flared position.
Figure 10 is a cross-sectional view of the embodiment of Figure 1 with the device in the flared position.
Figure 11 is an isometric view of the embodiment of Figure 1 after the device has been applied.
Figure 12 is a cross-sectional view of the embodiment of Figure 1 after the device has been applied.
Figure 13 is a cross-section view of the embodiment of Figure 2 with the device in the loaded position.
Figure 14 is an isometric view of the applier of Figure 2 with the device in the flared position.
Figure 15 is a cross-sectional view of the applier of Figure 2 with the device in the flared position.
Figure 16 is an isometric view of the applier of Figure 2 after the device has been applied.
Figure 17 is a cross-section view of the applier of Figure 2 after the device has been applied.
Figure 18 is a cross-sectional view of the applier of Figure 5 with the device in the loaded position.
Figure 19 is an isometric view of the path retainer of Figure 7 showing the relationship between the guide path and the guide clip when the device is being flared.
Figure 20 is a cross-sectional view of the applier of Figure 5 flaring the device.
Figure 21 is a cross-sectional view of the applier of Figure 5 with the device flared and restrained by the bullet nose.
Figure 22 is an isometric view of the path retainer of Figure 7 showing the relationship between the guide path and the guide clip when the device is flared and restrained by the bullet nose.
Figure 23 is a cross-sectional view of the applier of Figure 5 with the device being ejected.
Figure 24 is a cross-sectional view of the retainer of Figure 7 showing the relationship between the guide path and the guide clip when the device is being ejected.
Figure 25A is an isometric view of a distal end of an applier showing a bullet nose having a blunt surface.
Figure 25B is an isometric view of a distal end of an applier showing a bullet nose having a fluted surface.
Figure 25C is an isometric view of a distal end of an applier showing a bullet nose having a convex surface.
Figure 25D is an isometric view of a distal end of an applier showing a bullet nose having a concave surface.
Figure 25E is an isometric view of a distal end of an applier showing a bullet nose having an offset swept, asymmetric surface.
Figure 25F is an isometric view of a distal end of an applier showing a bullet nose having a spherical surface.

### Detailed Description of the Invention

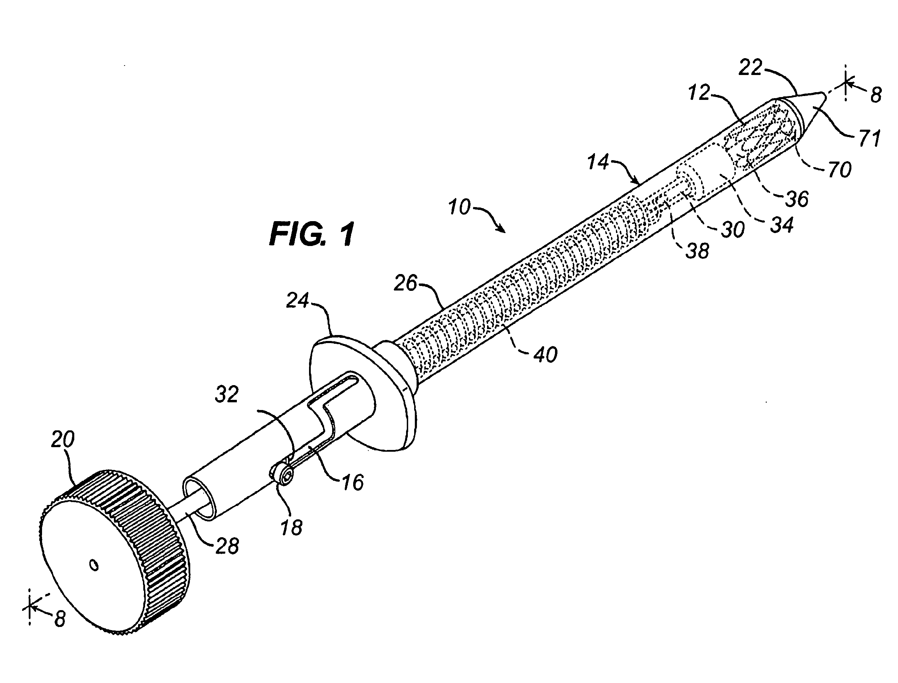

Figure 1 depicts an applier 10 configurable to apply an expandable medical device 12. Applier 10 has an enclosure, or a tube 14 for containing expandable medical device 12 and internal components of applier 10. Tube 14 may be created from a moldable plastic, and at least a portion of tube 14 may be translucent or transparent to allow visualization of device 12 or other internal components. Translucency or transparency could also allow light from a light source placed internal to tube 14 to pass externally to tube 14 to illuminate a work area. Guide slot 16 is positioned on tube 14 near the proximal end of tube 14. Guide slot 16 may have a shape to present stop positions or cam surfaces to the user to guide the user in proper placement of the components of applier 10 during actuation of applier 10. In applier 10 as depicted in Figure 1, the shape of guide slot 16 roughly approximates the letter "Z" and extends about at least a portion of the circumference of tube 14. A locator pin 18 moves within guide slot 16 and locates portions of components of applier 10. Figure 1 depicts locator pin 18 as a socket-head cap screw; however, locator pin 18 and guide slot 16 may comprise any cam-follower mechanism able to direct movement of components of applier 10. Knob 20 extends from the proximal end of applier 10 for control by the user. Tube handle 24 attaches to tube 14, also for grasping by the user to manipulate applier 10. A cap in the form of bullet nose 22 is placed at the distal end of tube 14. Bullet nose 22 protrudes from the distal end of tube 14, and has a distal end shaped to engage and dilate small stoma in tissue. This distal end shape may be conical, but could also be rounded, asymmetrical, pointed, or any shape to facilitate entry into tissue. Bullet nose 22 also possesses on its proximal surface a device taper 70. Device taper 70 is shown substantially conical in shape with the taper facing proximally towards expandable medical device 12 when expandable medical device 12 is loaded within applier 10. Device taper 70 may also take different surface shapes, such as, for example, a convex curved shape, which may facilitate flaring and deployment of expandable medical device 12.

Figure 1 further displays the internal mechanism 26 of applier 10 in isometric view. Pusher rod 28 extends distally from knob 20 and is hollow for at least a portion of its length to enclose other components. Pusher rod slot 30 opens into the internal portions of pusher rod 28 and is elongated to allow differential motion between pusher rod 28 and other components of internal mechanism 26, as will be seen. Pusher rod 28 possesses a guide bushing 32 near its proximal end to mate with tube 14 in such a way as to allow longitudinal movement and rotational movement between pusher rod 28 and tube 14. Locator pin 18 extends from guide bushing 32. A restraint element, called device pusher 34, is affixed to pusher rod 28 near the distal end of pusher rod 28, and restrains expandable medical device 12 from proximal movement when tube 14 moves proximally. Although device pusher 34 is shown with a substantially flat distal surface, device pusher 34 may alternately possess a tapered distal surface to assist in deploying device 12.

Bullet nose rod 36 is attached to and extends from bullet nose 22 proximally through pusher rod 28. A slip-fit clearance between bullet nose rod 36 and pusher rod 28 allows longitudinal movement between bullet nose rod 36 and pusher rod 28. Bullet nose rod slot 38 is cut into bullet nose rod 36 and aligns in operation with pusher rod slot 30.

Bullet nose rod 36 may take any of several configurations to facilitate manufacture and use. For example, bullet nose rod 36 may be integrally molded with bullet nose 22 to become one component. Alternatively, bullet nose rod 36 may have a threaded attachment to bullet nose 22 or may itself be composed of two components that are extendable for adjustability.

A force element embodied by spring 40 connects bullet nose rod 36 to tube handle 24 by engaging bullet nose rod slot 38. Spring 40 applies mechanical force urging bullet nose rod 36 and bullet nose 22 proximally. Since tube handle 24 affixes to tube 14, spring 40 applies mechanical force tending to hold bullet nose 22 and tube 14 together, but allowing relative displacement of bullet nose 22 away from tube 14 under a force opposing and greater than the force generated by spring 40.

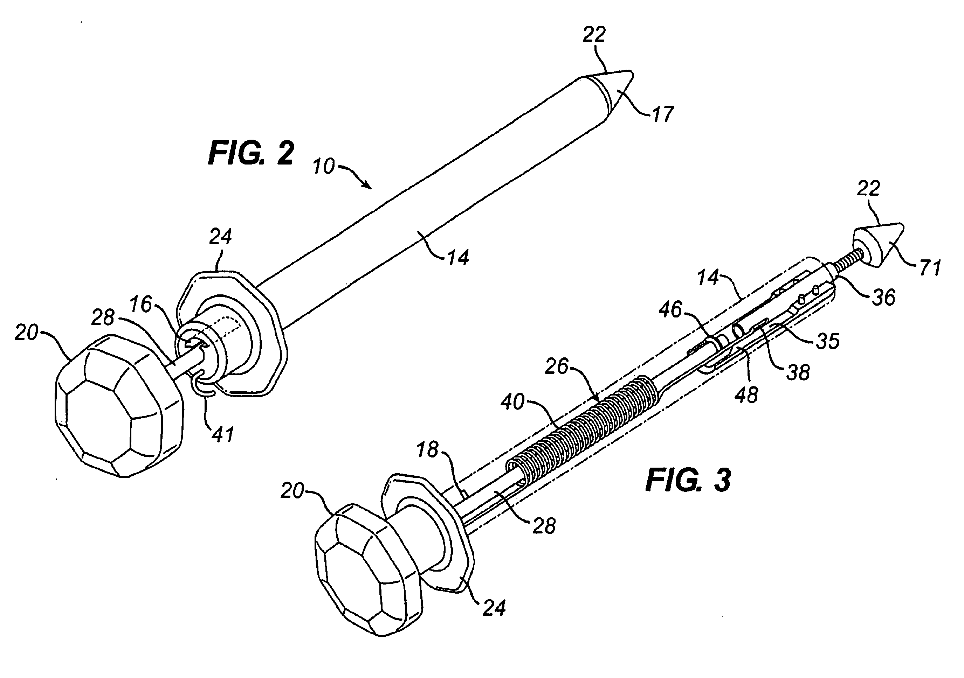

Figure 2 displays another embodiment of applier 10. As in the embodiment of applier 10 seen in Figure 1, tube handle 24 attaches at the proximal end of tube 14. However, in the embodiment of Figure 2, tube handle 24, rather than tube 14, carries guide slot 16. A leg 41 extending from spring 40 fastens to tube handle 24 through a small opening of tube handle 24, and may curve about a portion of tube handle 24 to locate it into position.

Figure 3 shows internal mechanism 26 of the embodiment of Figure 2. Tube 14 has been shown in phantom in Figure 3 for clarity, however, tube handle 24 remains to show positional relationships of components. Pusher rod 28 extends distally from knob 20 similarly to the embodiment of Figure 2. Device pusher half 35 locates at the distal end of pusher rod 28, affixed to pusher rod 28 by a snap ring 46. A second device pusher half 35, omitted for better visibility of associated components, mates with device pusher half 35 to enclose the distal end of pusher rod 28 and to form device pusher 34. A cover rod shown as bullet nose rod 36, extending from the cover shown as bullet nose 22, inserts into a distal end of device pusher 34. Clearance exists between device pusher 34 and pusher rod 28 so that longitudinal motion can occur between bullet nose rod 36 and device pusher 34. Bullet nose 22 protrudes from the distal end of bullet nose rod 36. As is possible in the embodiment of Figure 1, bullet nose 22 can combine with bullet nose rod 36 into one component, or bullet nose 22 can be a separate component attached to bullet nose rod 36 in a manner to allow an adjustable length between bullet nose 22 and bullet nose rod 36. The design can allow length adjustability by, for example, a threaded connection between bullet nose 22 and bullet nose rod 36. Also, as in the embodiment of Figure 1, bullet nose rod slot 38 is cut into the end of bullet nose rod 36. An embodiment of locater pin 18 extends radially from the surface of pusher rod 28 near the proximal side of pusher rod 28. Tube handle 24 attaches to the proximal end of tube 14.

Spring 40 hooks to bullet nose rod slot 38 to connect bullet nose rod 36 and tube handle 24. Spring 40 applies mechanical force urging bullet nose rod 36 and bullet nose 22 proximally. Since tube handle 24 affixes to tube 14, spring 40 applies mechanical force tending to hold bullet nose 22 and tube 14 together, but allowing relative displacement of bullet nose 22 away from tube 14 under an opposing force greater than that applied by spring 40. In the embodiment of Figure 3, spring 40 passes through a spring slot 48 created by the mating pusher halves 35.

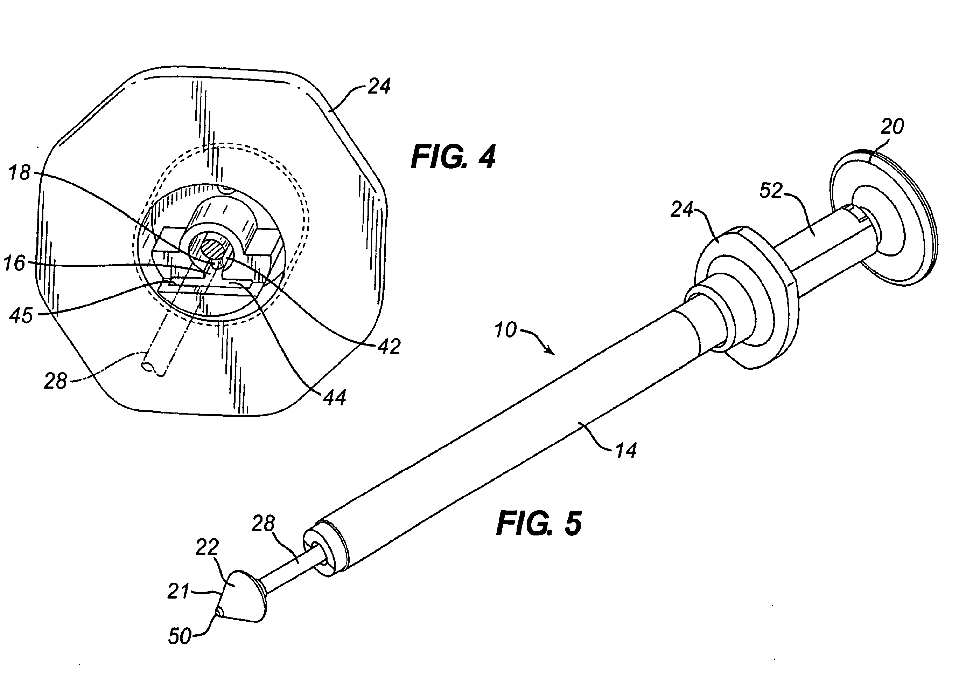

Figure 4 displays a view of tube handle 24 used in the embodiment of Figure 2. The view is taken from the distal end of tube handle 24. A guide slot 16 of the tube handle 24 shown in Figure 3 is also seen in Figure 4. Guide slot 16 comprises a longitudinal slot 42 following the axis of handle 24 at the proximal side of tube handle 24, opening into a horizontal slot 44 near the distal end of handle 24. A pin stop 45, in the embodiment of Figure 4, is a wall to prevent further distal movement of locator pin 18 without a preliminary rotational movement of pusher rod 28. Guide slot 16 has clearance for movement of locator pin 18 through longitudinal slot 42 and through horizontal slot 44, and so allows translation of locator pin 18 and rotation of locator pin 18 about the axis of pusher rod 28 when pusher rod 28 is rotated.

Figure 5 shows a third embodiment of applier 10. As in the other embodiments, applier 10 of Figure 5 carries a tube 14 with attached tube handle 24 at its distal end. Pusher rod 28 carries knob 20 at its proximal end and bullet nose 22 at its distal end. The embodiment of Figure 5 is shown with an access port 50 open through the entire length of applier 10. Other embodiments of applier 10 could have an access port 50 as well. Attached to tube 14 at the proximal end of tube 14 is path retainer 52, to direct movements of pusher rod 28, as will be seen.

Access port 50 may be used for placement of a tool to facilitate surgery. A surgeon may place, for example, through access port 50, a tool such as a guide wire to guide applier 10 within the body, a laser or surgical tool to effect further treatment, a fiber optic to give light to the surgical site, a fiber optic with an attached camera to visualize the surgical site, a wire to convey electrical energy, or a tube to convey pneumatic energy. Access port 50 may be sealed by, for example, an elastomeric plug when not in use if it becomes necessary to preclude passage of gas for reasons such as, for example, maintenance of pneumoperitenium.

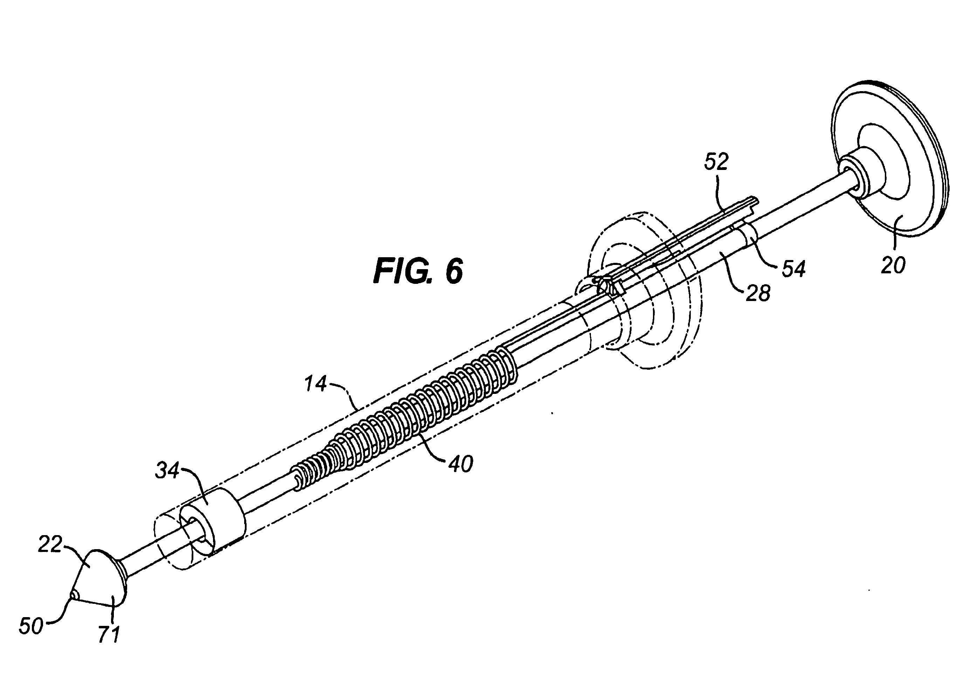

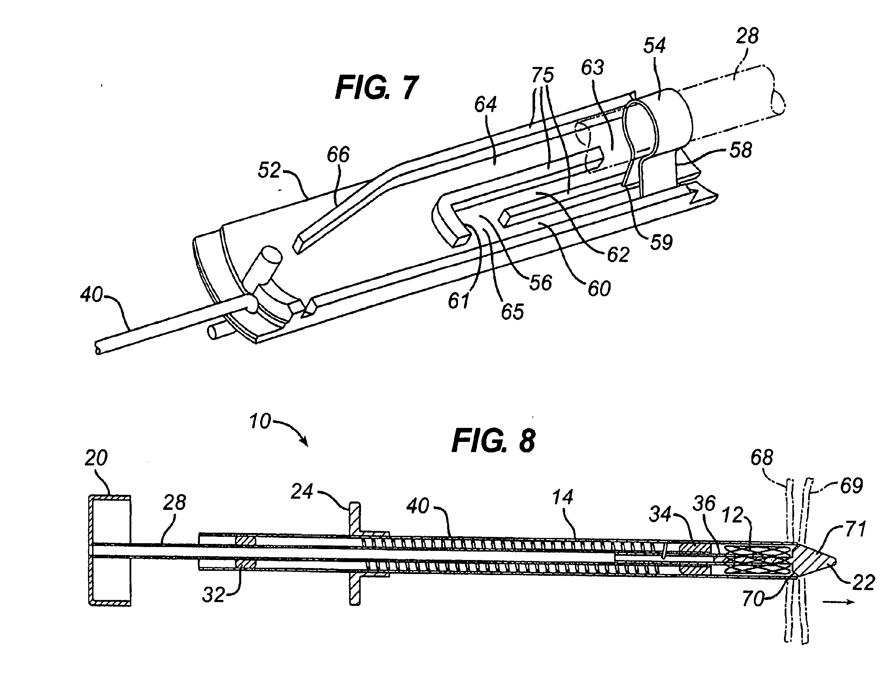

Figure 6 shows the embodiment of Figure 5 with tube 14 in phantom for clarity. In the embodiment shown in Figure 6, pusher rod 28 has knob 20 at its proximal end and bullet nose 22 at its distal end. Device pusher 34 attaches near the distal end of pusher rod 28. Spring 40 connects to tube 14 through path retainer 52, and to pusher rod 28; however, spring 40 could alternately attach directly to tube 14 or to any component affixed to tube 14. As in previous embodiments, spring 40 applies a force tending to urge bullet nose 22 towards tube 14, so that bullet nose 22 and tube 14 are engaged with a preload but can be deflected apart by a force large enough to overcome the preload. Guide clip 54 affixes to pusher rod 28 and engages path retainer 52 in a way to permit relative motion between guide clip 54 and path retainer 52. Guide clip 54, although affixed to pusher rod 28, has stationary guide clip leg 58 and deflectable guide clip leg 59 (Figure 7) to slidingly engage portions of path retainer 52. Stationary guide clip leg 58 remains substantially fixed relative to pusher rod 28, while deflectable guide clip leg 59 deflects outward, guided by cam surfaces on path retainer 52.

Figure 7 shows a close view of path retainer 52 used in the embodiment of applier 10 of Figure 5. This view shows the face of path retainer 52 that faces the interior of tube 14. The proximal end of spring 40 and positioning guide clip 54 are also shown in Figure 7. Path retainer 52 has a control path 56 to direct and control movement of pusher rod 28 through action of guide clip 54. Control path 56 consists of four paths for guidance and control of pusher rod 28. The four paths are flair path 60, return path 62, ejection path 64, and finish path 66. Rails 75 on the surface of path retainer 52 separate the paths. A flair stop 61 is at the distal end of return path 62, while clearance slot 63 opens between return path 62 and ejection path 64 near the proximal end of the paths. A distal clearance slot 65 opens between flair path 60 and return path 62 near the distal end of the paths. Stationary guide clip leg 58 and deflectable guide clip leg 59 are shown preloaded and compressed together by two of rails 75 to ride in flair path 60. In the relaxed state, stationary guide clip 58 and deflectable guide clip leg 59 spread to a distance to encompass the width between finish path 66 and flair path 60. In actuation of the mechanism, control path 56 permits consecutive relaxing and opening of deflectable guide clip leg 59 towards the relaxed, non-preloaded state. Stationary guide clip leg 58 remains within flair path 60, while deflectable guide clip leg 59 moves towards and into finish path 66.

Each embodiment of applier 10 has an operation sequence, as will be seen. The embodiments share commonality in that bullet nose 22 applies force generated by spring 40 to flair expandable medical device 12. When expandable medical device 12 is not extending beyond the distal end of tube 14, bullet nose 22 covers the end of tube 14 and is held in position by force applied by spring 40. When a force acts on bullet nose 22 and opposes the force of spring 40 and is greater than the force applied by spring 40, spring 40 deflects to allow bullet nose 22 to lift off of tube 40.

The embodiment of applier 10 shown in Figure 1 operates as shown in Figures 8 through 12. Figure 8 depicts the Figure 1 embodiment loaded with a medical device, such as an expandable medical device 12. Tube 14 contains expandable medical device 12. Expandable medical device 12 can be used to hold two tissue portions together to effect a therapeutic surgical treatment.

A surgeon can grasp applier 10 and place it into a patient's body. The surgeon can, for example, grasp the applier 10 by placing an index and middle finger on tube handle 24 and a thumb on knob 20. The surgeon maneuvers any embodiment of applier 10 to a portion of the body near organs needing surgical treatment, such as anastomosis. The surgeon may, for example, first divide a section of small intestine as a part of a medical procedure such as a gastric bypass operation. Or, the surgeon may anastomose bowel left behind by removal of a cancerous portion of bowel. The surgeon may create an otomy in a section of small intestine and extend applier 10 through the section of small intestine to a position where another otomy is desired. The surgeon may then make a second otomy in the wall of the same section and a third otomy in another section of intestine to be anastomosed. Applier 10 then can extend through the second and third otomies in the two sections of small intestine. The walls carrying the second and third otomies can be shown as proximal tissue portion 68 and distal tissue portion 69. Attaching these tissue portions can create an intestinal anastomosis when the tissue portions are portions of lumens of intestine. After extending applier 10 through proximal tissue portion 68 and distal tissue portion 69, a surgeon can operate applier 10 to effect a medical procedure such as an anastomosis.

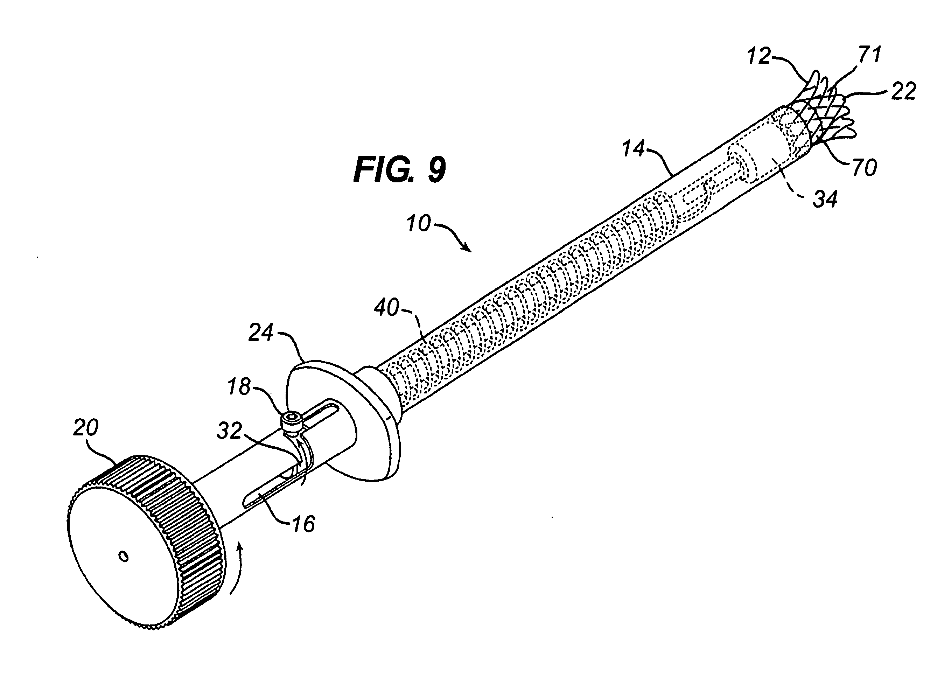

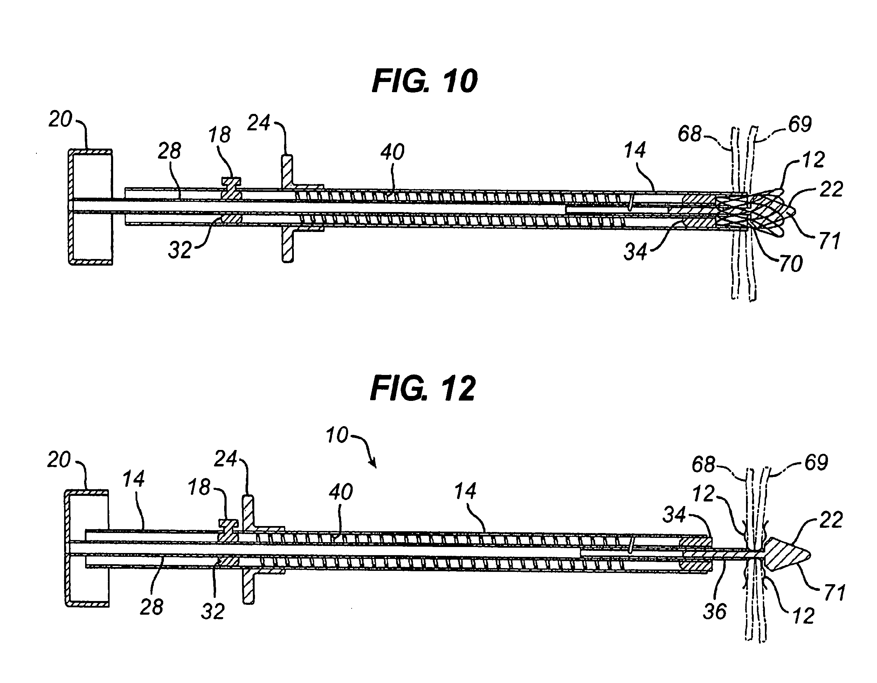

The surgeon urges tube handle 24 towards knob 20. Tube handle 24, affixed to tube 14, pulls tube 14 towards knob 20. Reactive force, placed by the surgeon on knob 20, transfers through pusher rod 28, to device pusher 34, to expandable medical device 12. As tube 14 slips proximally, device pusher 34 restrains any proximal movement of expandable medical device 12. Spring 40, connected between tube handle 24 and bullet nose rod 36, applies a force tending to pull bullet nose rod 36 in a proximal direction. With applier 10 in this first position when application is beginning, bullet nose rod 36 pulls bullet nose 22 proximally to maintain preloaded contact with tube 14, so that bullet nose 22 travels proximally with tube 14 as one unit relative to device pusher 34. Bullet nose rod 36 telescopes into the inner diameter of pusher rod 28. When bullet nose 22 reaches the distal end of expandable medical device 12, device taper 70 contacts expandable medical device 12 to flare it. Expandable medical device 12 applies a reactive force to bullet nose 22. When the distal reactive force overcomes the force applied by spring 40, the reactive force elongates spring 40 and separates bullet nose 22 slightly from tube 14. Flaring expandable medical device 12 begins to emerge from the opening created between tube 14 and bullet nose 22, as shown in Figures 9 and 10.

Figures 9 and 10 show flared expandable medical device 12 emerging from applier 10. As bullet nose 22 moves towards expandable medical device 12, device taper 70 presents a ramp to facilitate the flaring and opening of expandable medical device 12. Device taper 70 applies force to an internal portion of expandable medical device 12 to flare expandable medical device 12 and increase the diameter of the distal end of expandable medical device 12. In addition, the restraint that bullet nose 22 places on expandable medical device 12 prevents expandable medical device 12 from ejecting from tube 14 before expandable medical device 12 is placed into the correct position adjacent to tissue portions requiring surgical treatment. The surgeon can use the flared portion of expandable medical device 12, as it is captured by the force exerted by bullet nose 22, to pull distal tissue portion 69 towards proximal tissue portion 68 to effect treatment such as an anastomosis. Applier 10 with the extending, flared portion of captured expandable medical device 12 can be utilized as a tool to manipulate tissue.

As tube 14 moves towards knob 20, the proximal, axial portion of "Z"-shaped guide slot 16 moves relative to locator pin 18. When expandable medical device 12 is flared to the correct position for tissue manipulation, the circumferential portion of guide slot 16 abuts locator pin 18, preventing any further movement of tube 14 towards knob 20. To continue any further linear movement, the surgeon must now rotate tube 14 relative to locator pin 18.

Figure 9 shows that the circumferential portion of guide slot 16 moves relatively past locator pin 18. When the distal, axial portion of guide slot 16 aligns with locator pin 18, further movement of tube 14 towards knob 20 is permitted. After rotating tube 14 relative to knob 20 to produce the relative circumferential motion of guide slot 16 and locator pin 18, and placing distal tissue portion 69 and proximal tissue portion 68 near the distal end of applier 10 as shown in Figure 10, the surgeon is ready to eject expandable medical device 12 from applier 10.

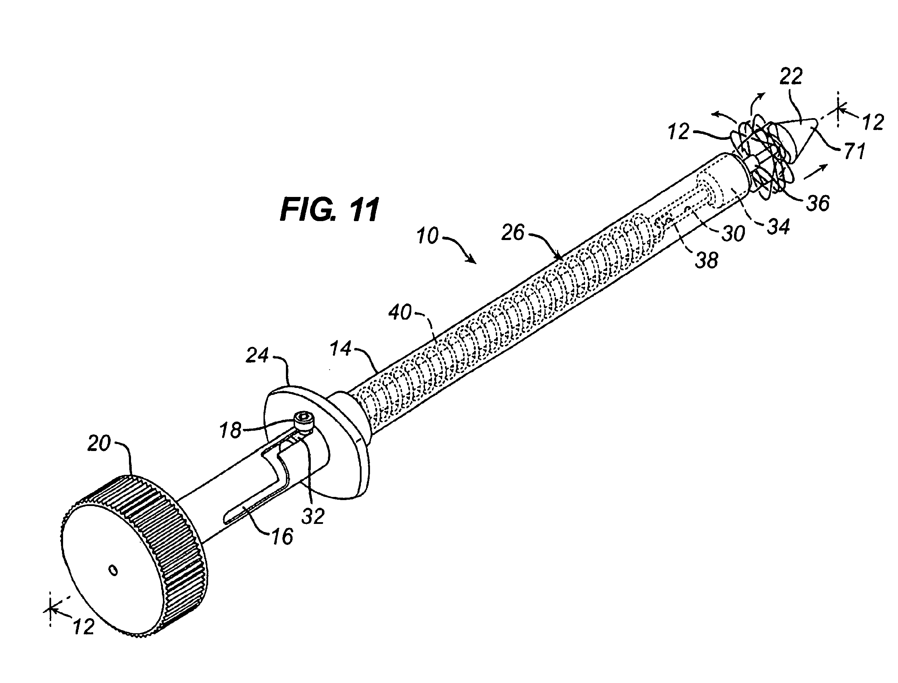

Figures 11 and 12 show the embodiment of applier 10 shown in Figure 1 with expandable medical device 12 ejected. To eject expandable medical device 12, the surgeon continues movement of tube 14 towards knob 20. Spring 40 continues to pull bullet nose 22 proximally, while bullet nose rod 36, attached to bullet nose 22, slides freely through pusher rod 28. Bullet nose 22 is still preloaded against flared expandable medical device 12, and expandable medical device 12 continues to emerge from the distal end of tube 14. When the proximal end of bullet nose rod slot 38, where spring 40 attaches, reaches the proximal end of pusher rod slot 30, force is now applied by spring 40 to pusher rod 28. Spring 40 now extends between pusher rod 28 and tube handle 24. Further proximal motion of tube 14 relative to pusher rod 28 extends spring 40. Spring 40 no longer preloads bullet nose 22 against expandable medical device 12. If the proximal end of bullet nose rod slot 30 moves proximal to the proximal end of pusher rod slot 38, force applied to the proximal end of bullet nose 22 by tissue portions or by expandable medical device 12 is no longer overcome by force from spring 40, so bullet nose 22 now is forced distally. There is no longer any relative motion between bullet nose 22 and pusher rod 28, so there is therefore no relative motion between bullet nose 22 and device pusher 34. However, in this later advanced position as expandable medical device 12 is nearing ejection, tube 14 moves relative to bullet nose 22 and device pusher 34. The space between device pusher 34 and bullet nose 22, containing expandable medical device 12, emerges from tube 14 to complete ejection of expandable medical device 12 as tube 14 is pulled proximally. Expandable medical device 12 assumes the correct orientation and geometry to perform a useful surgical procedure. The relative positions of components of applier 10 after ejection of expandable medical device 12 are shown in Figures 11 and 12.

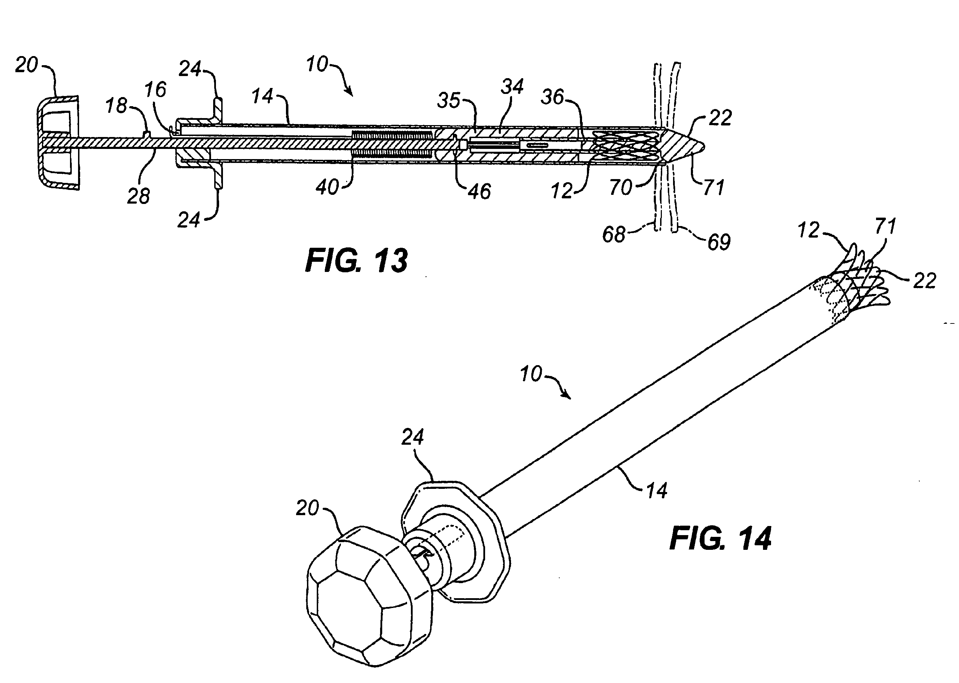

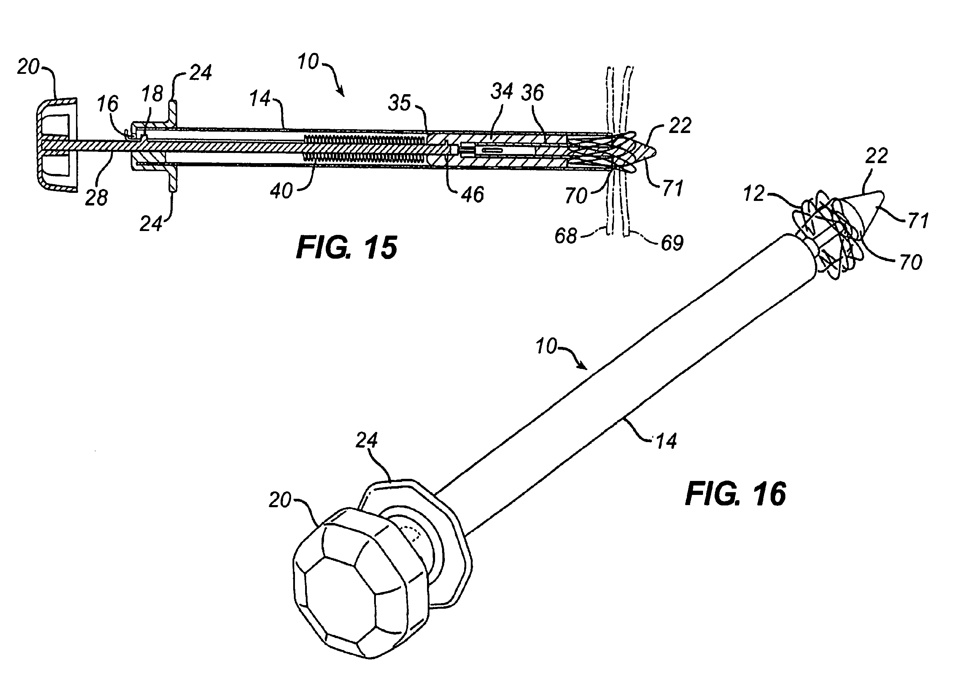

Figures 13 through 17 depict the operation of the embodiment of applier 10 shown in Figure 2. Figure 13 shows the embodiment of applier 10 loaded with expandable medical device 12. To begin to eject expandable medical device 12, the surgeon urges tube handle 24 towards knob 20. Tube handle 24, affixed to tube 14, pulls tube 14 towards knob 20. Reactive force placed by the surgeon on knob 20 transfers through pusher rod 28, snap ring 46, and device pusher 34 to expandable medical device 12. As tube 14 slips proximally, device pusher 34 restrains any rearward movement of expandable medical device 12. Simultaneously, force applied by spring 40, connected between tube handle 24 and bullet nose rod 36, urges bullet nose rod 36 proximally through device pusher 34. Bullet nose rod 36 pulls bullet nose 22 proximally to maintain preloaded contact with tube 14, so that tube 14 and bullet nose 22 proceed proximally as one unit. When bullet nose 22 reaches the distal end of expandable medical device 12, device taper 70 contacts expandable medical device 12 to flare it. Expandable medical device 12 applies a reactive force to bullet nose 22 greater than that applied by spring 40 to elongate spring 40 and separate bullet nose 22 slightly from tube 14. Flaring expandable medical device 12 begins to emerge from the opening created between tube 14 and bullet nose 22, as shown in Figure 14 and 15.

Figure 15 shows in section view flared expandable medical device 12 emerging from the embodiment of applier 10. Because spring 40 continues to urge bullet nose 22 proximally towards tube 14, device taper 70 presents a ramp to facilitate the flaring and opening of expandable medical device 12. In addition, the restraint that bullet nose 22 places on expandable medical device 12 prevents expandable medical device 12 from ejecting from tube 14 before expandable medical device 12 is placed into the correct position adjacent to tissue portions to be surgically treated. The surgeon can use the flared portion of expandable medical device 12, as it is captured by the force exerted by bullet nose 22, to pull a distal tissue portion 69 towards a proximal tissue portion 68 to effect treatment such as an anastomosis. Applier 10 with the extending, flared portion of captured expandable medical device 12 can be utilized as a tool to manipulate tissue during a surgical procedure.

As tube handle 24 moves proximally, guide slot 16 within tube handle 24 moves proximally relative to locator pin 18 through longitudinal slot 42. When expandable medical device 12 is flared to the correct position, pin stop 45 abuts locator pin 18, preventing any further movement of tube 14 towards knob 20. To continue any further linear movement, the surgeon must now rotate tube 14 relative to locator pin 18. Horizontal slot 44 (Figure 5) has clearance to allow this rotation. After locator pin 18 has rotated past pin stop 45, further movement of tube 14 towards knob 20 is permitted, and the surgeon is ready to completely eject expandable medical device 12 from applier 10.

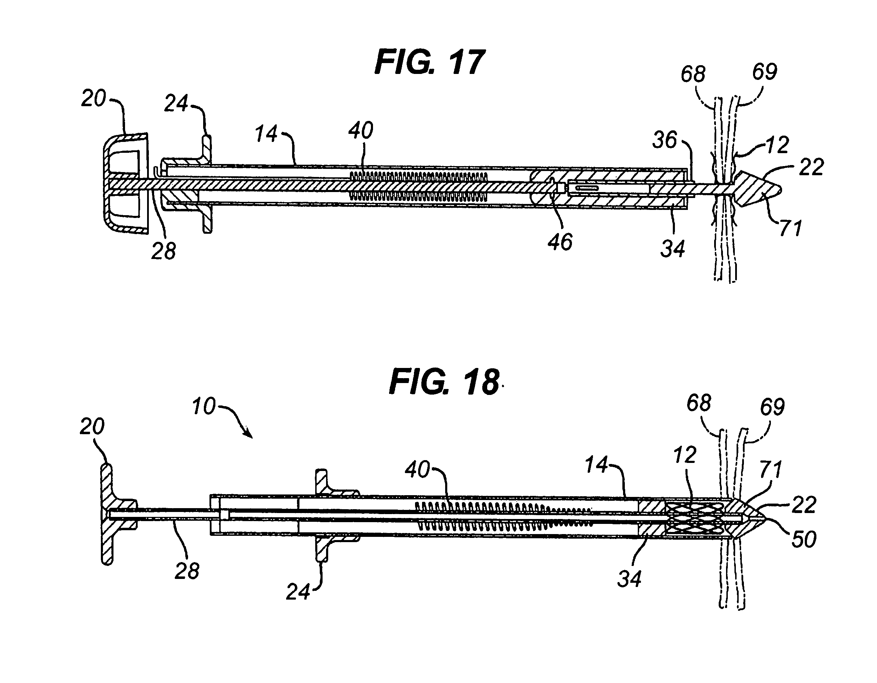

Figure 16 and 17 show cross-sectional views of expandable medical device 12 ejected from applier 10 and engaging proximal tissue portion 68 to distal tissue portion 69. To finish ejection of expandable medical device 12, the surgeon continues movement of tube 14 towards knob 20. Spring 40 continues to pull bullet nose 22 proximally, while bullet nose rod 36, attached to bullet nose 22, slides freely through device pusher 34. Bullet nose 22 is still preloaded against flared expandable medical device 12 by force exerted by spring 40. When the proximal end of bullet nose rod 36, where spring 40 attaches, reaches the distal end of pusher rod 28, force is now applied to pusher rod 28. Bullet nose 22 can no longer follow tube 14 proximally, and is no longer preloaded to tube 14. Further proximal motion of tube 14 relative to pusher rod 28 extends spring 40. There is no longer relative motion between bullet nose 22 and device pusher 34. Tube 14, however, moves relative to bullet nose 22 and device pusher 24. The space between device pusher 34 and bullet nose 22, containing expandable medical device 12, emerges from tube 14 to eject expandable medical device 12 as tube 14 is pulled proximally.

Figures 18 through 23 demonstrate the operation of the embodiment of applier 10 shown in Figure 5. Figure 18 shows the Figure 5 embodiment of applier 10 in the loaded position with expandable medical device 12 placed within tube 14. Force applied by spring 40 holds bullet nose 22 against the distal end of tube 14. A surgeon moves tube 14 proximally towards knob 20 against the force of spring 40 by grasping tube handle 24 as in previous embodiments. Tube 14 moves proximally relative to pusher rod 28 and device pusher 34. Device pusher 34 restrains proximal movement of expandable medical device 12. As tube 14 moves proximally, a gap opens between tube 14 and bullet nose 22. Expandable medical device 12 begins to emerge from the distal end of tube 14.

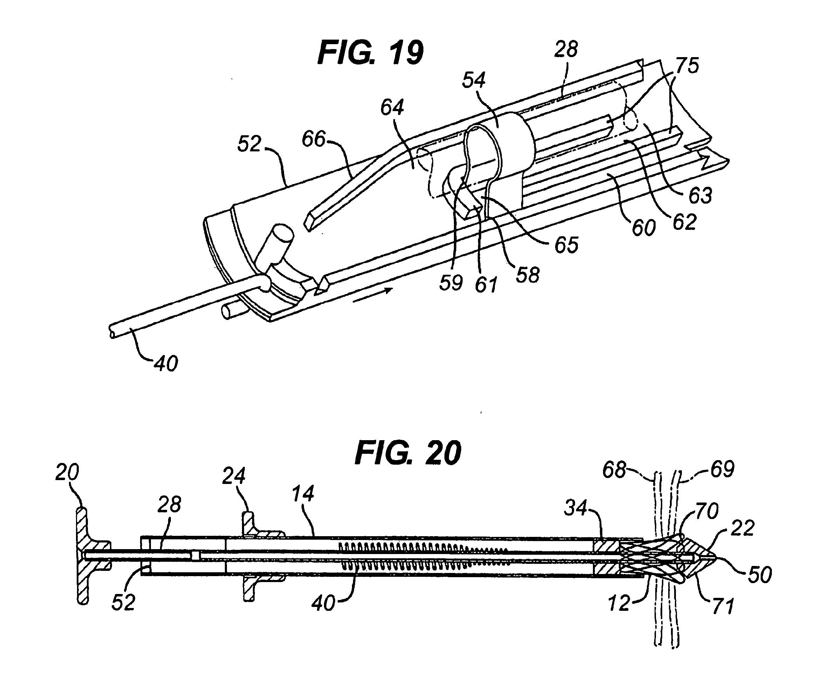

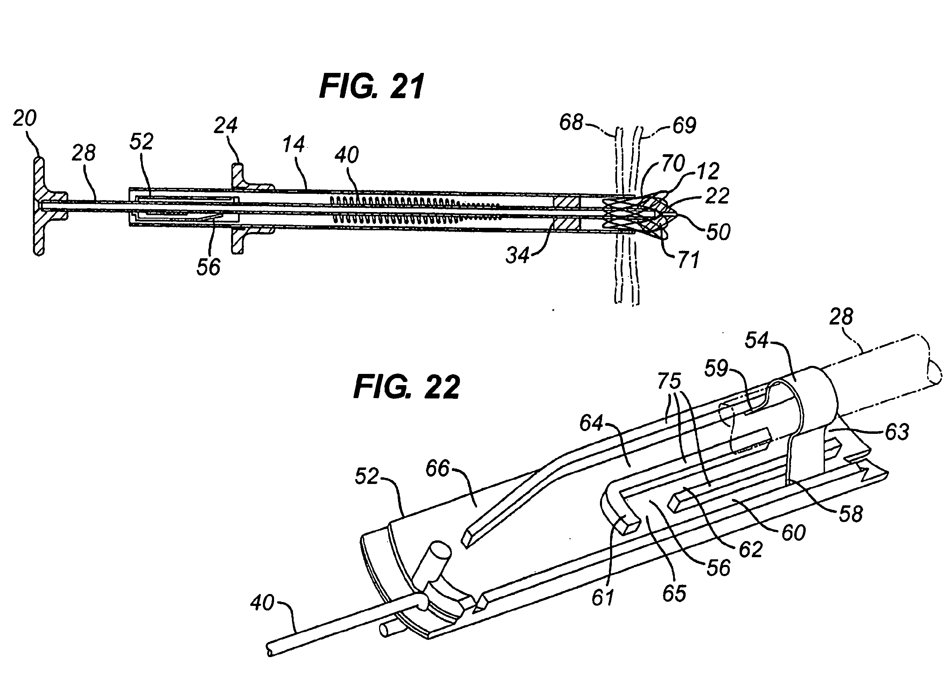

Figure 19 also shows an isometric view of path retainer 52 and guide clip 54. Path retainer 52 is attached to tube 14 and moves proximally with guide clip 54. As path retainer 52 moves proximally, control path 56 moves relative to guide clip 54 to control movements of tube 14. When tube 14 moves proximally from the position shown in Figure 18, flair path 60 moves past stationary guide clip leg 58 and deflectable guide clip leg 59 until flair stop 61 abuts guide clip 54. Flair stop 61 prevents further proximal motion of tube 14. When flair stop 61 abuts guide clip 54, the deflectable guide clip leg 59 will move through distal clearance slot 65 to return path 62. Applier 10 now takes the position shown in Figure 20, with expandable medical device 12 emerging from the distal end of tube 14. Releasing force from tube 14 will allow spring 40 to move tube 14 distally into the position shown in Figure 21.

Figure 21 shows a sectional view of the embodiment of applier 10 of Figure 6 with expandable medical device 12 flared from the distal end of tube 14. When the surgeon releases force from tube handle 24, spring 40 pulls tube 14 distally towards bullet nose 22. Device taper 70 contacts expandable medical device 12 to assist in flaring expandable medical device 12. Force from spring 40 is applied to device 12.

This embodiment of applier 10 in the configuration of Figure 21, much like previous embodiments, may be used to capture distal tissue wall 69 to approximate it to a proximal tissue wall to perform a surgical procedure such as an anastomosis. A surgeon can use applier 10 with the flared expandable medical device 12 as a tool to manipulate tissue.

Figure 22 shows path retainer 52 and guide clip 54 as applier 10 is in the flared position of Figure 21. Path retainer 52 has moved to the initial position relative to guide clip 54 while deflectable guide clip leg 59 was within return path 62. Deflectable guide clip leg 59 then moved through clearance slot 63 to ejection path 64 when path retainer 52 reached the initial position with guide clip 54 at its proximal end.

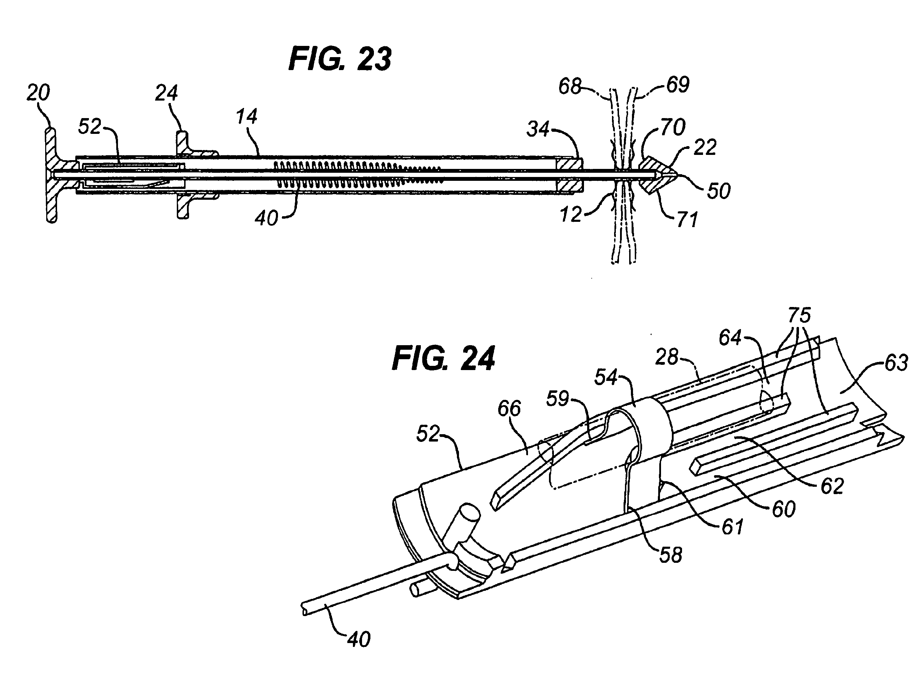

Figure 23 shows expandable medical device 12 ejected by applier 10. After expandable medical device 12 has been placed into position, the surgeon may now eject device 12 by pulling tube handle 24 towards knob 20 to again urge tube 14 proximally relative to pusher rod 28. When device pusher 34 contacts the proximal end of expandable medical device 12, expandable medical device 12 is prevented from further proximal movement. Tube 14 moves proximally from expandable medical device 12 to deploy it.

Figure 24 depicts the action of path retainer 52 moving proximally past guide clip 54 while applier 10 deploys expandable medical device 12. Ejection path 64 moves proximally past deflectable guide clip leg 59. As there exists no stop at the distal end of ejection path 64, tube 14 can move further proximally to a position allowing device pusher 34 to completely eject expandable medical device 12. The curve of guide rail 75 adjacent to ejection path 64 places a side force on deflectable guide clip leg 59, moving it closer to stationary guide clip leg 58. The force generated by moving deflectable guide clip leg 59 can give tactile feedback to the surgeon that tube 14 is approaching the end of its allowable proximal movement. After tube 14 has reached its most proximal position and device 12 has been ejected, the surgeon may now release any force pulling tube handle 24 towards knob 20. Spring 40 will pull tube 14 distally relative to knob 20, while finish path 66 moves distally past deflectable guide clip leg 59. Applier 10 has ejected expandable medical device 12.

One will recognize that these descriptions of movement of portions of applier 10 describe relative movement of the elements with respect to each other. For example, movement of tube 14 proximally towards knob 20 can also be described as movement of knob 20 distally towards tube 14. As another example, movement of tube 14 proximally past device pusher 34 could also be described as distal movement of device pusher 34 within a stationary tube 14. In the latter example, expandable medical device 12 is described as restrained from proximal movement by device pusher 34 when tube 14 moves proximally past. Changing the stationary element from device pusher 34 to tube 14 changes the description to one of expandable medical device 12 urged distally by distally advancing device pusher 34.

It will be recognized that equivalent structures may be substituted for the structures illustrated and described herein and that the described embodiment of the invention is not the only structure that may be employed to implement the claimed invention. As one example of an equivalent structure that may be used in the present invention, hydraulics, electronics, or pneumatics may be used to move tube 14 relative to device pusher 34. Computer control could be used with electronics and a feedback loop to move tube 14 and to selectively tension a force element based on the amount of tissue force applied. As a further example of an equivalent structure that may be used in the present invention, robotics could be used with applier 10 attached to a controlled robotic arm that moves the mechanism of applier 10 to effect an anastomosis. Robotics would allow a surgeon distant from the surgical site to perform a procedure.

As a further example of an equivalent structure, tube 14 could become a flexible tube, and the mechanisms within applier 10 may become flexible to maneuver through a long lumen, such as a section of small bowel, to effect an anastomosis through a long, flexible lumen. Such a long, flexible tube may be used laproscopically or endoscopically.

As a farther example of an equivalent structure, applier 10 could have a long, rigid, curved tube, or a long, rigid, straight tube, and applier 10 could be placed through an obturator port and used laproscopically or endoscopically. Length and curvature becomes advantageous in endoscopic or laproscopic surgery, especially when performing a surgical procedure on a bariatric patient. In either a rigid or a flexible form of an applier 10, restriction of gas flow through the instrument becomes advantageous when maintenance of a pneumoperiteneum is desired as in, for example, endoscopic surgery.

As a further example of an equivalent structure that may be used in the present invention, applier 10 may have a geometry small enough to be conveniently placed through the opening of a hand port used for hand-assisted laproscopic surgery, such as, for example, the Lap-Disk^{®} hand port sold by Ethicon Endo-Surgery in Cincinnati, Ohio. A surgeon using applier 10 through a hand port may use an endoscope through a secondary port for visualization, and may also maintain a pneumoperiteneum. The surgeon may also make use of trocars, graspers, cutters, and other endoscopic instruments inserted through auxiliary ports to assist in grasping lumens or creating otomies in lumens to perform surgical procedures such as anastomosis.

As a further example of an equivalent structure that may be used in the present invention, a long, rigid version of applier 10, or a long, flexible embodiment of applier 10 may be used through an auxiliary port while tissue is manipulated by the surgeon using a hand placed through a hand port.

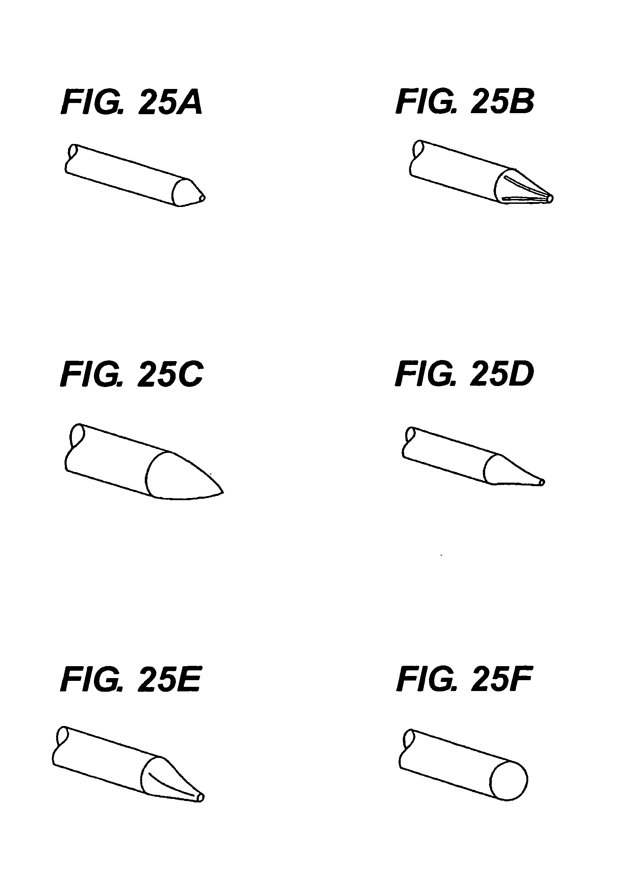

As other examples of equivalent structures, the surface of distal taper 71 on bullet nose 22 may take many forms advantageous for various types of tissue manipulation, as illustrated in Figures 25A through 25F. Figure 25A represents a conical tipped nose that is blunted for low tissue trauma and for good visibility past the distal end. Figure 25B depicts a nose that is fluted to allow torque to be applied to tissue. Figure 25B depicts four flutes, although three or any other number of flutes may suffice. Figure 25C depicts a nose having a convex curve for rapid dilation of an otomy in a short space, while Figure 25D shows a nose having a concave surface for gentle dilation of friable tissue. An offset swept nose, shown in Figure 25E, may be used because of its asymmetry for better visibility to one side and may be used to assist in manipulation by using its asymmetry to minimally grasp tissue. Figure 25F shows a spherical nose to produce a short length for operation in limited space and to reduce the chance of tissue trauma. Combinations of these surfaces may also be advantageous, for example, a nose having a concave surface as depicted in Figure 25D may also posses flutes as depicted in Figure 25B. Other combinations may occur to one skilled in the art.

While preferred embodiments of the present invention have been shown and described herein, it will be obvious to those skilled in the art that such embodiments are provided by way of example only. Numerous variations, changes, and substitutions will now occur to those skilled in the art without departing from the invention. Accordingly, it is intended that the invention be limited only by the scope of the appended claims.

## Claims

1. An applier (10) for a surgical procedure to tissue, said applier comprising:
a. a tube (14) having a distal end, a proximal end and a longitudinal axis therebetween;
b. a pusher rod (28) having distal and proximal ends, said pusher rod having a cap (22) attached to its distal end and a restraint element (34) disposed thereon proximal to said cap, said pusher rod is disposed within said tube such that said proximal end of said pusher rod is proximal to said proximal end of said tube, and said cap is distal to said distal end of said tube and wherein said restraint element is disposed within said tube;
c. an expandable medical device (12) disposed within said distal end of said tube between said restraint element and said cap, **characterised in that** said cup is spring-biased against said distal end of said tube.

2. The applier of claim 1 wherein said restraint element abuts against an inner surface of said tube.

3. The applier of claim 1 wherein said cap and said restraint element are to move longitudinally with respect to each other.

4. An applier according to Claim 3 further comprising a means for preventing distal movement of said restraint element toward said cap after said restraint element has initially moved distally a predetermined distance.

5. An applier according to Claim 1 wherein at least a portion of said tube is transparent.

6. An applier according to Claim 1 wherein said cap tapers distally to form a cone.

7. An applier according to Claim 1 wherein said restraint element tapers distally to form a cone.

8. An applier according to Claim 1 wherein said pusher rod has open distal and proximal ends with a lumen extending therethrough.

## Patentansprüche

1. Applikator (10) für einen chirurgischen Eingriff an Gewebe, wobei der Applikator umfasst:
a. eine Röhre (14), die eine distales Ende, ein proximales Ende und eine longitudinale Achse dazwischen aufweist;
b. einen Schiebestab (28), der ein distales und ein proximales Ende aufweist, wobei der Schiebestab eine Kappe (22), die an seinem distalen Ende befestigt ist, und ein Sicherungselement (34) aufweist, das sich darauf proximal zur Kappe befindet, wobei der Schiebestab innerhalb der Röhre derart angeordnet ist, dass das proximale Ende des Schiebestabes proximal zu dem proximalen Ende der Röhre liegt und sich die Kappe distal zu dem distalen Ende der Röhre befindet, wobei sich das Sicherungselement innerhalb der Röhre befindet;
c. eine expandierbare medizinische Einrichtung (12), die innerhalb des distalen Endes der Röhre zwischen dem Sicherungselement und der Kappe angeordnet ist, **dadurch gekennzeichnet, dass** die Kappe mit einer Feder gegen das distale Ende der Röhre vorgespannt ist.

2. Applikator nach Anspruch 1, **dadurch gekennzeichnet, dass** das Sicherungselement an die Innenfläche der Röhre angrenzt.

3. Applikator nach Anspruch 1, **dadurch gekennzeichnet, dass** die Kappe und das Sicherungselement zum longitudinalen Aufeinanderzubewegen dienen.

4. Applikator nach Anspruch 3, ferner umfassend ein Mittel zum Verhindern distaler Bewegung des Sicherungselements zu der Kappe, nachdem sich das Sicherungselement anfänglich eine vorab festgelegte Strecke distal bewegt hat.

5. Applikator nach Anspruch 1, **dadurch gekennzeichnet, dass** wenigstens ein Teil der Röhre transparent ist.

6. Applikator nach Anspruch 1, **dadurch gekennzeichnet, dass** sich die Kappe distal verjüngt, um einen Kegel zu bilden.

7. Applikator nach Anspruch 1, **dadurch gekennzeichnet, dass** sich das Sicherungselement distal verjüngt, um einen Kegel zu bilden.

8. Applikator nach Anspruch 1, **dadurch gekennzeichnet, dass** der Schiebestab offene distale und proximale Enden hat, durch die sich ein Lumen erstreckt.

## Revendications

1. Applicateur (10) pour une procédure chirurgicale sur un tissu, ledit applicateur comprenant :
a. un tube (14) ayant une extrémité distale, une extrémité proximale et un axe longitudinal entre celles-ci ;
b. une tige-poussoir (28) ayant des extrémités distale et proximale, ladite tige-poussoir ayant un capuchon (22) fixé à son extrémité distale et un élément de retenue (34) disposé sur celle-ci à proximité dudit capuchon, ladite tige-poussoir est disposée à l'intérieur dudit tube de telle manière que ladite extrémité proximale de ladite tige-poussoir se trouve à proximité de ladite extrémité proximale dudit tube, et ledit capuchon se trouve à distance de ladite extrémité distale dudit tube et dans lequel ledit élément de retenue est disposé à l'intérieur dudit tube ;
c. dispositif médical expansible (12) disposé à l'intérieur de ladite extrémité distale dudit tube entre ledit élément de retenue et ledit capuchon, **caractérisé en ce que** ledit capuchon est dévié par un ressort contre ladite extrémité distale dudit tube.

2. Applicateur selon la revendication 1, dans lequel ledit élément de retenue bute contre une surface interne dudit tube.

3. Applicateur selon la revendication 1, dans lequel ledit capuchon et ledit élément de retenue se déplacent longitudinalement l'un par rapport à l'autre.

4. Applicateur selon la revendication 3, comprenant en outre des moyens destinés à prévenir le mouvement distal dudit élément de retenue vers ledit capuchon après que ledit élément de retenue s'est initialement déplacé de manière distale sur une distance prédéterminée.

5. Applicateur selon la revendication 1, dans lequel au moins une partie dudit tube est transparente.

6. Applicateur selon la revendication 1, dans lequel ledit capuchon s'effile distalement pour former un cône.

7. Applicateur selon la revendication 1, dans lequel ledit élément de retenue s'effile distalement pour former un cône.

8. Applicateur selon la revendication 1, dans lequel ladite tige-poussoir a des extrémités distale et proximale ouvertes avec une lumière s'étendant entre celles-ci.
